(19)

Europäisches
Patentamt
European
Patent Office
Office européen
des brevets

(11) **EP 3 879 539 A1**

(12) **EUROPEAN PATENT APPLICATION**

(43) Date of publication:
**15.09.2021 Bulletin 2021/37**

(21) Application number: **21161553.9**

(22) Date of filing: **09.03.2021**

(51) Int Cl.:
**G16H 20/70** (2018.01)          **G16H 50/20** (2018.01)
**G16H 50/30** (2018.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.03.2020 IN 202021010921**

(71) Applicant: **Tata Consultancy Services Limited 400021 Mumbai, Maharashtra (IN)**

(72) Inventors:
• **Krishnan, Balasubramaniam**
  **560009 Karnataka (IN)**
• **Raghavan, Venkatachari**
  **600113 Tamil Nadu (IN)**
• **Vijayakumar, Arun**
  **682042 Kerala (IN)**
• **Muralidharan, Kartik**
  **560066 Karnataka (IN)**
• **Ghose, Avik**
  **700156 Kolkata, West Bengal (IN)**
• **Duggirala, Mayuri**
  **411013 Pune, Maharashtra (IN)**

(74) Representative: **Goddar, Heinz J.**
**Boehmert & Boehmert**
**Anwaltspartnerschaft mbB**
**Pettenkoferstrasse 22**
**80336 München (DE)**

(54) **SYSTEM AND METHOD OF DETERMINING PERSONALIZED WELLNESS MEASURES ASSOCIATED WITH PLURALITY OF DIMENSIONS**

(57)      A processor implemented method of determining personalized wellness measures associated plurality of dimensions of an individual is provided. The processor implemented method includes at least one of: receiving, plurality of information associated with plurality of sensors; processing, the plurality of information associated with the plurality of sensors to obtain plurality of low-level features; determining, at least one digital behavioral marker based on the plurality of low-level features; processing, at least one dimension associated with a plurality of well-being of the individual based on the at least one digital behavioral marker to determine a set of objective features; generating, one or more wellness scores for the set of objective features associated with the at least one well-being dimension of the individual; and dynamically recommending, one or more behavior changes based on the one or more generated wellness scores of the individual.

receiving, via one or more hardware processors, plurality of information associated with plurality of sensors 302

processing, via the one or more hardware processors, the plurality of information associated with the plurality of sensors to obtain a plurality of low-level features 304

determining, via the one or more hardware processors, at least one digital behavioral marker based on the plurality of low-level features 306

processing, via the one or more hardware processors, at least one dimension associated with a plurality of well-being of the individual based on the at least one digital behavioral marker to determine a plurality of self-reported analyzed data 308

processing, via the one or more hardware processors, the at least one dimensions through (i) a plurality of high-level features, and (ii) the plurality of self-reported analyzed data to identify a set of objective features associated with at least one well-being dimension of the individual 310

generating, via the one or more hardware processors, one or more wellness scores for the set of objective features associated with the at least one well-being dimension of the individual 312

dynamically recommending, via the one or more hardware processors, one or more behavior changes based on the one or more generated wellness scores of the individual 314

**FIG. 3**

EP 3 879 539 A1

**Description**

CROSS-REFERENCE TO RELATED APPLICATIONS AND PRIORITY

The present application claims priority from Indian patent application no. 202021010921, filed on March 13, 2020. The entire contents of the aforementioned application are incorporated herein by reference.

TECHNICAL FIELD

The disclosure herein generally relates to wellness management, and, more particularly, to system and method of determining personalized wellness measures associated with plurality of dimensions.

BACKGROUND

Recently, there has been an increasing body of research investigating use of mobile and wearable devices as a tool to measure and compute well-being. For example, several mobile solutions are proposed to utilize a self-monitoring and intervention-based treatment of depression. There has been significant thought process across multiple fields including philosophy, medicine, psychology, sociology, and socioeconomics, the concept of well-being remains elusive. While the definition remains unclear, common patterns can be identified. First, the dimensions of well-being include mental, emotional, physical, social, material, and professional. Second, while essentially a subjective assessment, both subjective and objective measurement methodologies exist. Third, the scope of well-being is predominantly long-term, with a few weeks a usual time scale in subjective self-assessments. While existing systems through technologies attempt to capture and measure one or more of the well-being dimensions what is missing however is the ability to measure these dimensions whilst factoring additional contextual parameters to provide a more personalized wellness measure.

The research approach adopted by these solutions can be explained with an example to capture objectively measure-able behavioral markers such as location and social interaction and correlate them with one of the wellness conditions like depression. Such digital behavioral markers have been defined as higher-level features reflecting behaviors, cognition, and emotions, which are measured using low-level features and sensor data collected from digital technology, including mobile and wearable computing devices. The discovery of such significant correlations between objective features and depressive mood symptoms has raised great enthusiasm regarding using mobile and wearable devices in the monitoring of a person's well-being. For example, if the measured objective feature deviates from healthy behavior, an alarm or trigger could be raised. However, it is not easy to identify which objective features consistently correlate with depressive mood symptoms and how they influence them. Some studies have shown similar results, while others have shown contradicting results. For example, a statistically significant negative correlation between the number of outgoing SMS text messages and a Hamilton depression rating scale (HDRS) are found, whereas another approach found a statistically significant positive correlation. Well-being refers to balance between positive emotions and moods such as happiness, contentment and self-confidence, absence of negative emotions like anxiety and depression as well as aspects such as life fulfilment, life satisfaction and resilience to deal with and recover from setbacks.

SUMMARY

Embodiments of the present disclosure present technological improvements as solutions to one or more of the above-mentioned technical problems recognized by the inventors in conventional systems. For example, in one aspect, a processor implemented method of determining personalized wellness measures associated plurality of dimensions of an individual is provided. The processor implemented method includes at least one of: receiving, via one or more hardware processors, plurality of information associated with plurality of sensors; processing, via the one or more hardware processors, the plurality of information associated with the plurality of sensors to obtain a plurality of low-level features; determining, via the one or more hardware processors, at least one digital behavioral marker based on the plurality of low-level features; processing, via the one or more hardware processors, at least one dimension associated with a plurality of well-being of the individual based on the at least one digital behavioral marker to determine a plurality of self-reported analyzed data; processing, via the one or more hardware processors, the at least one dimensions through (i) a plurality of high-level features, and (ii) the plurality of self-reported analyzed data to identify a set of objective features associated with at least one well-being dimension of the individual; generating, via the one or more hardware processors, one or more wellness scores for the set of objective features associated with the at least one well-being dimension of the individual; and dynamically recommending, via the one or more hardware processors, one or more behavior changes based on the one or more generated wellness scores of the individual.

In an embodiment, the plurality of low-level features may correspond to information associated with at least one of (i) social, (ii) physical activity, (iii) location, (iv) inputs from physiological parameters of the end user and (iv) device. The processor implemented method may further includes, identifying, via the one or more hardware processors, at least one triggering condition and at least one suitable intervention as a function of the plurality of high-level features. In an embodiment, a subjective measurement of wellness may be based on a contextual knowledge and at least one appropriate weight is assigned to calculate a type of wellness. In an embodiment, the root cause of underlying condition in terms of wellness may be identified to provide nudging recommendations to the user. In an embodiment, prediction and prognosis of underlying condition may be performed against population in comparison to provide one or more recommendations associated with at least one wellness measures to the user. In an embodiment, personalization of wellness may be through flexibility of defining one or more types of wellness to calculate overall wellness of the user and to provide inputs through longitudinal analysis of specific activity.

In another aspect, there is provided a processor implemented system to determine personalized wellness measures across plurality of dimensions of an individual. The system comprises a memory storing instructions; one or more communication interfaces; and one or more hardware processors coupled to the memory via the one or more communication interfaces, wherein the one or more hardware processors are configured by the instructions to: receive, plurality of information associated with plurality of sensors; process, the plurality of information associated with the plurality of sensors to obtain a plurality of low-level features; determine, at least one digital behavioral marker based on the plurality of low-level features; process, at least one dimension associated with a plurality of well-being of the individual based on the at least one digital behavioral marker to determine a plurality of self-reported analyzed data; process, the at least one dimensions through (i) a plurality of high-level features, and (ii) the plurality of self-reported analyzed data to identify a set of objective features associated with at least one well-being dimension of the individual; generate, one or more wellness scores for the set of objective features associated with the at least one well-being dimension of the individual; and dynamically recommend, one or more behavior changes based on the one or more generated wellness scores of the individual.

In an embodiment, the plurality of low-level features may correspond to information associated with at least one of (i) social, (ii) physical activity, (iii) location, (iv) inputs from physiological parameters of the end user and (iv) device. In an embodiment, the one or more hardware processors may be further configured to identify at least one triggering condition and at least one suitable intervention as a function of the plurality of high-level features. In an embodiment, a subjective measurement of wellness may be based on a contextual knowledge and at least one appropriate weight is assigned to calculate a type of wellness. In an embodiment, the root cause of underlying condition in terms of wellness may be identified to provide nudging recommendations to the user. In an embodiment, prediction and prognosis of underlying condition may be performed against population in comparison to provide one or more recommendations associated with at least one wellness measures to the user. In an embodiment, personalization of wellness may be through flexibility of defining one or more types of wellness to calculate overall wellness of the user and to provide inputs through longitudinal analysis of specific activity.

In yet another aspect, there are provided one or more non-transitory machine-readable information storage mediums comprising one or more instructions which when executed by one or more hardware processors causes at least one of: receiving, plurality of information associated with plurality of sensors; process, the plurality of information associated with the plurality of sensors to obtain a plurality of low-level features; determining, at least one digital behavioral marker based on the plurality of low-level features; processing, at least one dimension associated with a plurality of well-being of the individual based on the at least one digital behavioral marker to determine a plurality of self-reported analyzed data; processing, the at least one dimensions through (i) a plurality of high-level features, and (ii) the plurality of self-reported analyzed data to identify a set of objective features associated with at least one well-being dimension of the individual; generating, one or more wellness scores for the set of objective features associated with the at least one well-being dimension of the individual; and dynamically recommending, one or more behavior changes based on the one or more generated wellness scores of the individual.

In an embodiment, the plurality of low-level features may correspond to information associated with at least one of (i) social, (ii) physical activity, (iii) location, (iv) inputs from physiological parameters of the end user and (iv) device. In an embodiment, the one or more hardware processors may be further configured to identify at least one triggering condition and at least one suitable intervention as a function of the plurality of high-level features. In an embodiment, a subjective measurement of wellness may be based on a contextual knowledge and at least one appropriate weight is assigned to calculate a type of wellness. In an embodiment, the root cause of underlying condition in terms of wellness may be identified to provide nudging recommendations to the user. In an embodiment, prediction and prognosis of underlying condition may be performed against population in comparison to provide one or more recommendations associated with at least one wellness measures to the user. In an embodiment, personalization of wellness may be through flexibility of defining one or more types of wellness to calculate overall wellness of the user and to provide inputs through longitudinal analysis of specific activity.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates a system for determining personalized wellness measures associated plurality of dimensions of an individual, according to embodiments of the present disclosure.
FIG. 2 is an exploded view of the exemplary system illustrates determination of personalized wellness measures associated plurality of dimensions of the individual, according to embodiments of the present disclosure.
FIG. 3 is an exemplary flow diagram illustrating a method of determining the personalized wellness measures associated plurality of dimensions of the individual, according to embodiments of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments. It is intended that the following detailed description be considered as exemplary only, with the true scope being indicated by the following claims.

There is a need for a platform that can assist automatically in identifying objective measures best suited in computing the different dimensions that constitutes a person's well-being. Further the platform should abstract the complexities in defining and computing the various high-level objective measures using low-level features and sensor data. Embodiments herein provides a system and method of computing personalized wellness measures associated plurality of dimensions.

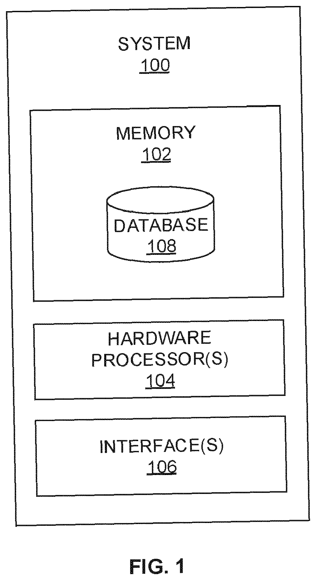

Referring now to the drawings, and more particularly to FIGS. 1 through 3, where similar reference characters denote corresponding features consistently throughout the figures, there are shown preferred embodiments and these embodiments are described in the context of the following exemplary system and/or method.

FIG. 1 illustrates a system for determining personalized wellness measures associated plurality of dimensions of the individual, according to embodiments of the present disclosure. In an embodiment, the system 100 includes one or more processors 104, communication interface device(s) or input/output (I/O) interface(s) 106, and one or more data storage devices or memory 102 operatively coupled to the one or more processors 104. The memory 102 comprises a database 108. The one or more processors 104 that are hardware processors can be implemented as one or more microprocessors, microcomputers, microcontrollers, digital signal processors, central processing units, state machines, logic circuitries, and/or any devices that manipulate signals based on operational instructions. Among other capabilities, the processor(s) is configured to fetch and execute computer-readable instructions stored in the memory. In an embodiment, the system 100 can be implemented in a variety of computing systems, such as laptop computers, notebooks, hand-held devices, workstations, mainframe computers, servers, a network cloud and the like.

The I/O interface device(s) 106 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. In an embodiment, the I/O interface device(s) can include one or more ports for connecting a number of devices to one another or to another server.

The memory 102 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. In an embodiment, the memory 102 includes a plurality of modules and a repository for storing data processed, received, and generated by the plurality of modules. The plurality of modules may include routines, programs, objects, components, data structures, and so on, which perform particular tasks or implement particular abstract data types.

Further, the database 108 stores information pertaining to inputs fed to the system 100 and/or outputs generated by the system 100 (e.g., data/output generated at each stage of the data processing), specific to the methodology described herein. More specifically, the database 108 stores information being processed at each step of the proposed methodology.

The repository, amongst other things, includes a system database and other data. The other data may include data generated as a result of the execution of one or more modules in the plurality of modules. The database 108 may store information but are not limited to, information associated with at least one of: (i) one or more parameters associated with a user, (ii) one or more dimensions, and (iii) one or more sensors. Further, the database 108 stores information pertaining to inputs fed to the system 100 and/or outputs generated by the system (e.g., at each stage), specific to the methodology described herein. More specifically, the database 108 stores information being processed at each step of the proposed methodology.

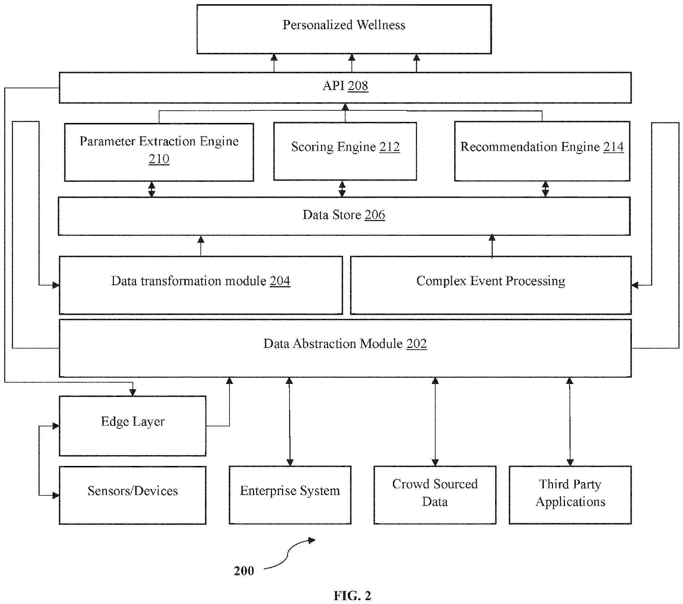

FIG. 2 is an exploded view of the exemplary system 200 illustrates determination of personalized wellness measures associated plurality of dimensions of the individual, according to embodiments of the present disclosure. The system 200 provides an exemplary personalized wellness platform which includes a data abstraction module 202, data transformation module 204, a data store 206, a data application programming interface (API) 208, a parameter extraction engine 210, a scoring engine 212, and a recommendation engine 214. In an embodiment, the personalized wellness platform corresponds to a persona-based wellness platform. The data abstraction module 202 is configured to abstract data from one or more sources including sensors, one or more devices, customer relationship management (CRM), other external/internal enterprise systems and crowd sources systems. In an embodiment, the data abstraction module 202 is configured to expose one or more low-level features and a sensor data that are used by a high-level feature module to define the various digital markers. The high-level feature module is configured to define and compute one or more digital behavioral markers or objective features as a function of the one or more low-level features and the sensor data. In an embodiment, the individual corresponds to a user, a patient.

In an embodiment, a sample set of low-level features are computed using a combination of the available one or more sensors and one or more device accessible parameters. For example, the sample set of low-level features are listed below:

a) **Social:** Features describing social behavior, including activity related to phone calls, texting, social network size, and other people in the user's context.

Call duration (incoming or outgoing)-Call log, Call frequency (incoming or outgoing)-Call log, Calls missed-Call log, Maximum call duration-Call log, Number of conversations-Call log, SMS text messages received (characters)-SMS text message log, Characters in SMS text message (sent or received)-SMS text message log, SMS text message (sent or received)-SMS text message log, Speak duration-Call log, and Devices seen-Bluetooth.

b) **Physical activity:** Features describing physical activity, including movement and step count.

Activity (afternoon, day, evening, morning, night)-Accelerometer, Autocorrelation-Accelerometer, Vigorous activity -Accelerometer, Distance-Accelerometer- GPS, Energy expenditure-Multiple sensors, Fourier analysis-Accelerometer, Inactivity duration-Accelerometer, Jerk-Accelerometer, Movement duration-GPS, Movement speed-Accelerometer-GPS, Movement speed variance-GPS, RMSSD-Accelerometer, Sample Entropy-Accelerometer, SD of stillness-Accelerometer, and Steps-Accelerometer, Pedometer.

c) **Location:** Features describing mobility, including GPS tracking, clustering of location (e.g., home stay), and transition time.

Cell tower ID-GSM, Home stay-GPS, Location clusters-GPS, Break duration-FM radio signal, Circadian rhythm-GPS, Entropy-GPS, Home to location cluster-GPS, Maximum distance between clusters-GPS, Raw entropy-GPS, Routine index-GPS, Transition time-GPS, Location variance-GPS, and Coverage area-GPS. **d) Device:** Features describing device (mobile phone or wearable) usage, including app usage, lock or unlock events, and classification of app usage.

Communication or social usage-App, Duration-App, Browser usage-App, Images taken-Camera, Number of running apps-App, Response time-Notification, Screen active duration or frequency-Screen, Screen clicks-Screen, Time from arrival till seen-Notification, and Time from seen till acted.

The wellness platform allows through suitable interfaces to define the objective or high-level features as a function of the low-level information. For example, a depression severity, an objective measure, can be defined as a function of number of SMS text received, screen clicks and location variance. In an embodiment, thresholds can be set for these variables allowing for a scaled measurement of depression severity. In an embodiment, the system allows for defining the well-being dimension. For example, high emotional well-being can be defined as a function of low. depression severity, medium, physical activity and so on.

In one embodiment, the one or more devices corresponds to one or more medical devices. In another embodiment, the one or more devices corresponds to one or more wearable devices. The data transformation module 204 is configured to standardize one or more data formats and enriching data with context and business knowledge. A data transformation layer is configured to hold responsibility of transforming the data into unified format and store in the data store 206 considering the application include process information for variety of the sensors and the one or more devices. In an embodiment, contextualization and business enrichment of the data is also performed as part for transformation. In an embodiment, apart from objective measurement of type of wellness, subjective measurement of wellness includes contextual knowledge and appropriate weights to calculate one or more type of wellness (based on the type of wellness that individual wants to choose).

The data store 206 is configured to store data in standardized format. The data application programming interface (API) 208 is configured to share the data as APIs. In an embodiment, one or more personas is abstracted and one or more attributed are defined for one or more personas. The parameter extraction engine 210 is configured to extract one or more derived parameters.

The one or more derived parameters corresponds to but not limited to are:

a) Basic Parameters, Persona information like Name, Address, Age, Height, Location, Weight, Profession, Interest, Habits etc.
b) Physical and Physiological information like Pulse, Blood Pressure, Skin color, Skin Temperature, Skin Resistance, Heart Rate.
c) Activity Parameters, walking speed, Calorie, Activities performed for daily living.
d) Social, Emotional, Intellectual, Environment, and Behavioral.

In one embodiment, the system is configured to collect pertinent information regarding subject behavioral activities including: a) psychological parameters, for example, anxiety, depression, psychosocial crisis, suicidal ideation, and/or stress levels; b) physical parameters, for example, nutritional intake, and exercise extent; and c) a plurality of qualitative and quantitative parameters, for example, social engagement, mood, effectiveness, motivation, and commitment levels.

In one embodiment, the system is configured to combine knowledge of personal health information with consistent behavioral patterns to help a subject to identify personalized behavior-changing objectives. For example, one or more medical information describing the subject's history of anxiety and high stress levels are considered to provide objectives and recommendations towards reducing anxiety and stress levels, such as getting a minimum of seven hours of sleep a night, maintaining healthy eating habits, participating in physical recreational activities, limiting intake of alcohol, and scheduling regular psychotherapy sessions, and medical checkups.

In an exemplary embodiment, a depression severity, an objective measure, can be defined as a function of number of SMS text received, screen clicks and location variance. In an embodiment, thresholds can be set for these variables allowing for a scaled measurement of depression severity. The system 200 allows defining the well-being dimension of the subject. For example, 'high' emotional well-being can be defined as a function of 'low' depression severity, 'medium' physical activity and so on. Considering, one must note however that individuals vary in their phone usage. Some users may use SMS as a messaging option more than others. The system 200 is configured to check whether a particular objective measure identified is suitable for that corresponding individual. The system 200 provides analytic support to identify whether the well-being definition and corresponding one or more constituents which suits for the individual. In an embodiment, the exemplary personalized wellness platform is flexible to add or delete any parameter at any point and time. The scoring engine 212 is configured to generated one or more score based on one or more customized algorithms. The recommendation engine 214 is configured to nudge behavior changes based on wellness score of the user.

In an exemplary embodiment, a method of for computing personalized wellness measures associated plurality of dimensions by considering but not limited to quantify the whole human wellness from with complete contextual relevance to the individual level rather than comparing to similar other individuals at the demographic level. The process involves but not limited to:

a) Biological medical needs and recommended values of physical and physiological needs of the consumer;
b) Wellness Survey as perceived by the individual;
c) Cross Sectional - Multiple contextual life assessment as per needs of the customer's wellness and sample parameters:

i. Social - Automate social wellness by calculating time spent in talking to others and by calculating quality of discussions.
ii. Emotional - through Questionnaire i.e., purpose of life and meaning, engaging work.
iii. Physical i.e., through Cardiovascular fitness.
iv. Intellectual i.e., capability to resolve conflicts.
v. Spiritual i.e., Self-Reflection.
vi. Psychological i.e., Stress.
vii. Occupational i.e., Confident about my career decisions.
viii. Environmental i.e., Safety of location.
ix. Comparison of Wellness against similar personas i.e., anonymous profiles.

x. Effectiveness of taking various parameters for calculating wellness claim.

xi. Assigning of weights determined by individual with recommendations provided from various models.

In an embodiment, based on the customer's preference of including one or above parameters of wellness based on context, customer's wellness parameters are measured. The system 200 provides a various recommended level from various established model of wellness for his/her to compare his/her scores. In an embodiment, contextual knowledge of assigning weights to particular activity which is contributing to disease or underlying condition/state (e.g., social isolation) is made possible in the personalized wellness platform in order to have contextualized inputs for different types of wellness. For example, like monitoring capability to understand how important like SMS messaging (as the person may not use messaging for communicating with others) is for wellness program and social isolation.

In and embodiment, scoring of wellness includes one or more following pre-defined steps before actuals calculation. There are two types of parameters but not limited to which can impact the score are:

i. Derived information: Stress, Anxiety, Aggression, Happiness, self-reflection, safety etc.

ii. Direct Information: walking speed, Calorie burned, Pulse, Heart Rate, Blood Pressure, cortisol and adrenalin etc.

In an embodiment, a mathematic technique like weighted average are performed and alternatively statistical methods like t-test are performed and combination thereof.

For example, the derived information is as mentioned below:

Considering, scoring pulse using t-test.

H0: Pulse is normal = 75

HI: Pulse for adults of age between 35 and 45 is not normal as <> 75 By calculating the sample mean pulse measured every minute of an individual Mr. X of age 37 for period of 15 mts = 78

$$\text{t-test} = (\text{sample mean} - \text{population mean})/[\text{standard deviation}/\text{sqrt}(n)]$$

n = 15

df= 15 -1 =14

Sample = {76,75,78,78,79,77,78,79,80,78,78,79,78,79,78}

Stddev = 1.211

T-test = (78-75)/ 1.211 = 2.478

Degree of freedom = 14

T value = 1.761

The calculated value is more than table value hence we reject H0. Hence pulse of Mr. X is not normal and have impact on stress which again impact wellness. Let the impact score be X with weightage N, then the stress impact could be, $(n_1 x_1 + n_2 x_2 + N X + n_4 x_4 + n x_1) / (n_1 x_1 + n_1 + n_1 + n_1 + n_1)$ if applying weighted average method.

Considering, estimated healthcare services account for just 10% of longevity, while social and environmental factors account for twice that at 20%, genetics 30%, and individual behaviors an estimated 40%. The persona-based wellness platform is looking at contributing components of holistic wellness which account for healthcare expenses related to one disease condition say BP/Hypertension. The persona-based wellness platform originates from a complicated interaction of genes and several environmental risk factors including aging, smoking, lack of exercise, overweight and obesity, elevated salt intake, stress, depression, and anxiety.

In an embodiment, to measure and indicate external and derived components of wellness which are impacting healthcare, a holistic wellness measurement system's questionnaire and one or more sensors at home and sensors at wearables device are configured to track derived parameters through individual behaviors, through questionnaire for parameters like social, environmental, Occupational and related it with direct measurements from a blood pressure (BP).

For example, the questionnaire could be given appropriate weightage of 0 and 1 based on whether if the factor is present or not in individual:

a) Being fired from one's job i.e., Occupational Wellness.

b) Having a child going out of home for studies i.e., Emotional.

c) Dealing with the death of a loved person i.e., Emotional.

d) Getting divorced i.e., Emotional.

e) Suffering an injury i.e., Environmental.

f) Experiencing money related issues i.e., Social.

In an embodiment, a logistic regression is performed to solve root cause and provide nudging recommendations.

H0: social wellness, occupational wellness impact BP positively.
HI: social wellness and occupational wellness does not impact BP positively.

The logistic regression can be used to predict the wellness and components of wellness which are impacting potential diseases condition like hypertension. In an embodiment, the wellness platform involve one or more statistical techniques like 't' value and a logistic regression and corresponding machine learning techniques to identify one or more root causes (i.e., type of wellness which is impacting the user the most for a specific underlying condition) and provide nudging recommendations.

A probability of hypertension increasing, for every additional unit decline in occupational wellness (being laid off from job), emotional wellness (dealing with death of loved one), can measure how the BP is being affected and how much increase in BP is identified. Dependent variable of the external factors (like emotional, occupational or social) can be tested using logistic regression - log-likelihood.

Log-Likelihood (LL) can be represented as:

$$P(Y) = 1/ 1+\exp{}^{\wedge} - (b_0+b_1x_1+b_2x_2+b_3x_3+\ldots b_nx_n).$$

$$Deviance = -2 * Log\ Likelihood.$$

Here the parameters:

x1 represents emotional wellness,
x2 represents social wellness,
x3 represents occupational wellness.

P(Y) is probability of different type of wellness affecting blood pressure positively. Similarly, b0 is the constant and b1, b2.and bn are various co-efficient which are impacting the weight with which each type of wellness impacting the deterioration of hypertension.

Hence multiple correlations of higher decline in emotional wellness factor impacting hypertension and corresponding weight of impact can be attributed using the above equation. In an embodiment, modelling using Maximum Likelihood estimation (MLE) in Machine learning algorithm to find the probability and impact of each factor.

The personalized wellness platform support to find out Maximum Likelihood estimation (MLE) if this can give a prediction in terms of how soon a person is likely to fall into potentially hypertension of higher band by comparison of population which shows similar characteristics.

In an embodiment, can also be further taken up for Post Hoc Test to identify which parameters of wellness are contributing to higher increase in BP using Poet Hoc testing and hence identified parameters like say for example if occupational wellness is found to be highest contributor in the form of external factor for hypertension, then a nudge can be given to individual to fix the occupational wellness in order to contain the hypertension.

In an embodiment, Cox and Snell's R^2 value of model can be correlated to build the model with higher correlation to calculate it for wider population and use this as a basis of machine learning for predicting similar occurrences in population. For example, the Cox & Snell's presents the R-squared as a transformation of the -21n[L(MIntercept)/L(MFull)] statistic).

The personalized wellness platform is configured to include an individual nudge his/her behavior and environments so that his/her other aspects of wellness are tweaked in order to have better impact of BP and hence better overall wellness.

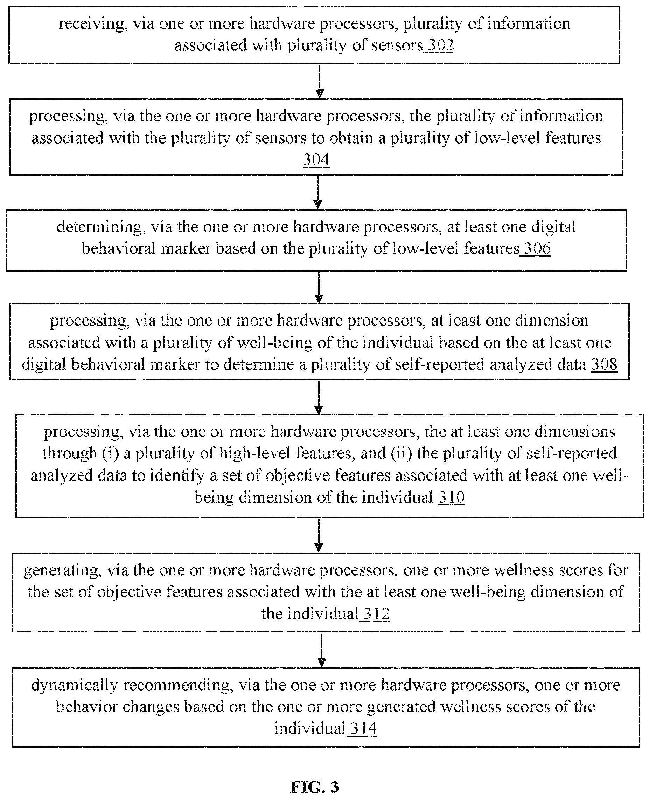

FIG. 3 is an exemplary flow diagram illustrating a method of determining personalized wellness measures associated plurality of dimensions of the individual according to embodiments of the present disclosure. In an embodiment, the system 100 comprises one or more data storage devices or the memory 102 operatively coupled to the one or more hardware processors 104 and is configured to store instructions for execution of steps of the method by the one or more processors 104. The flow diagram depicted is better understood by way of following explanation/description. The steps of the method of the present disclosure will now be explained with reference to the components of the system 200 as depicted in FIG. 2.

At step 302, a plurality of information associated with plurality of sensors is received. At step 304, the plurality of information associated with the plurality of sensors are processed to obtain a plurality of low-level features. In an embodiment, the plurality of low-level features may correspond to information associated with at least one of (i) social, (ii) physical activity, (iii) location, (iv) inputs from physiological parameters of the end user and (iv) device. At step 306, at least one digital behavioral marker is determined based on the plurality of low-level features. At step 308, at least one dimension associated with a plurality of well-being of the individual is processed based on the at least one digital behavioral marker to determine a plurality of self-reported analyzed data. At step 310, the at least one dimensions is processed through (i) the plurality of high-level features, and (ii) the plurality of self-reported analyzed data to identify a set of objective features associated with at least one well-being dimension of the individual. At step 312, one or more wellness scores is generated for the set of objective features associated with the at least one well-being dimension of the individual. At step 314, one or more behavior changes is recommended dynamically based on the one or more generated wellness scores of the individual.

In an embodiment, personalization through identification of high-level features (e.g., type of wellness) relevant to an individual that is contextual to the user is supported. In an embodiment, the individual can be allowed to define the type of wellness he/she would like to monitor for his/her holistic wellness program. For example, if person is not interested in occupational wellness as he/she does not work, spiritual wellness then that particular type of wellness is given low/no weightage based on the one or more cases. The processor implemented method at least one triggering condition and at least one suitable intervention as a function of the plurality of high-level features may be identified. In an embodiment, a subjective measurement of wellness may be based on a contextual knowledge and at least one appropriate weight is assigned to calculate a type of wellness. In an embodiment, the root cause of underlying condition in terms of wellness may be identified to provide nudging recommendations to the user. In an embodiment, prediction and prognosis of underlying condition may be performed against population in comparison to provide one or more recommendations associated with at least one wellness measures to the user. In an embodiment, personalization of wellness may be through flexibility of defining one or more types of wellness to calculate overall wellness of the user and to provide inputs through longitudinal analysis of specific activity.

The embodiments of present disclosure herein address unresolved problem of computing personalized wellness measures across one or more dimensions. The embodiments of the present disclosure provide an information platform which abstract the wellness parameters in a unified platform after contextual enrichment. The embodiments of the present disclosure abstract the complexity in obtaining the data, from one or multiple devices, necessary to compute the different well-being dimensions. The embodiments of the present disclosure include a feature to measure wellness in terms of customizable wellness parameters on multiple dimensions such as Social, Emotional, Physical, Intellectual, Spiritual, Psychological, Occupational, Environmental that can be customized to the individual. The platform abstracts one or more complexities of obtaining sensor data as well as provides access to additional context information necessary in computing a personalized wellness measure. Further, the platform provides flexibility of defining the wellness measures within each dimension as well as setting triggering criteria and suitable interventions.

The embodiments of the present disclosure provide a personalized wellness scores well defined in terms of average of various dimensions of wellness. The embodiments of the present disclosure provide an ability to recalibrate individual wellness measurement beyond one or more basic parameters. The embodiments of the present disclosure are a comparison between external monitoring and self-monitoring and indication to monitor environment to adapt itself to one or more wellness needs. The embodiments of the present disclosure provide an ability to sense through combination of wearable sensors, sensors at home and other designated approved monitoring places. The personalized wellness platform includes a feature of questionnaire and behavioral in sensing in place which gives a well-rounded score of wellness than the wellness from unique sensors alone.

The personalized wellness platform includes a feature to measure customized form of wellness in terms of customizable wellness parameters on multiple dimensions like - Social, Emotional, Physical, Intellectual, Spiritual, Psychological, Occupational, Environmental that can be customized to the individual. The embodiments of the present disclosure overcome limitation of going beyond basic definitions of wellness like physical, mental and emotional in smart environments by environments adjusting itself to monitor the wellness as defined by the individual. The embodiments of the present disclosure in which the individual chooses to select the right form of wellness after showing him/her demographic averages on various forms of wellness. The embodiments of the present disclosure in which the personalized wellness platform provide a unified data model to store variety of wellness parameters. The personalized wellness platform can abstract various wellness parameters from various sources including sensors and devices. The personalized wellness platform provides a gamification and recommenders for continuous and sustained engagements. The personalized wellness platform provides an ability to compare across demographics and category.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1. A processor implemented method of determining personalized wellness measures associated plurality of dimensions of an individual, comprising:

   receiving, via one or more hardware processors, plurality of information associated with plurality of sensors;
   processing, via the one or more hardware processors, the plurality of information associated with the plurality of sensors to obtain a plurality of low-level features;
   determining, via the one or more hardware processors, at least one digital behavioral marker based on the plurality of low-level features;
   processing, via the one or more hardware processors, at least one dimension associated with a plurality of well-being of the individual based on the at least one digital behavioral marker to determine a plurality of self-reported analyzed data;
   processing, via the one or more hardware processors, the at least one dimensions through (i) a plurality of high-level features, and (ii) the plurality of self-reported analyzed data to identify a set of objective features associated with at least one well-being dimension of the individual;
   generating, via the one or more hardware processors, one or more wellness scores for the set of objective features associated with the at least one well-being dimension of the individual; and
   dynamically recommending, via the one or more hardware processors, one or more behavior changes based on the one or more generated wellness scores of the individual.

2. The processor implement method as claimed in claim 1, wherein the plurality of low-level features corresponds to information associated with at least one of (i) social, (ii) physical activity, (iii) location, (iv) inputs from physiological parameters of the end user and (iv) device.

3. The processor implement method as claimed in claim 1, further comprising, identifying, via the one or more hardware processors, at least one triggering condition and at least one suitable intervention as a function of the plurality of high-level features.

4. The processor implement method as claimed in claim 1, wherein a subjective measurement of wellness based on a contextual knowledge and at least one appropriate weight is assigned to calculate a type of wellness.

5. The processor implement method as claimed in claim 1, wherein the root cause of underlying condition in terms of wellness can be identified to provide nudging recommendations to the user.

6. The processor implement method as claimed in claim 1, wherein prediction and prognosis of the underlying condition are performed against population in comparison to provide one or more recommendations associated with at least one wellness measures to the user.

7. The processor implement method as claimed in claim 1, wherein personalization of wellness through flexibility of defining one or more types of wellness to calculate overall wellness of the user and to provide inputs through longitudinal analysis of specific activity.

8. A system (100) to determine personalized wellness measures across plurality of dimensions of an individual, comprising:

   a memory (102) storing instructions;
   one or more communication interfaces (106); and
   one or more hardware processors (104) coupled to the memory (102) via the one or more communication interfaces (106), wherein the one or more hardware processors (104) are configured by the instructions to:

      receive, plurality of information associated with plurality of sensors;
      process, the plurality of information associated with the plurality of sensors to obtain a plurality of low-level features;
      determine, at least one digital behavioral marker based on the plurality of low-level features;
      process, at least one dimension associated with a plurality of well-being of the individual based on the at least one digital behavioral marker to determine a plurality of self-reported analyzed data;
      process, the at least one dimensions through (i) a plurality of high-level features, and (ii) the plurality of self-reported analyzed data to identify a set of objective features associated with at least one well-being dimension of the individual;
      generate, one or more wellness scores for the set of objective features associated with the at least one well-being dimension of the individual; and
      dynamically recommend, one or more behavior changes based on the one or more generated wellness scores of the individual.

9. The system (100) as claimed in claim 8, wherein the plurality of low-level features corresponds to information associated with at least one of (i) social, (ii) physical activity, (iii) location, (iv) inputs from physiological parameters of the end user and (iv) device.

10. The system (100) as claimed in claim 8, wherein the one or more hardware processors is further configured to identify at least one triggering condition and at least one suitable intervention as a function of the plurality of high-level features.

11. The system (100) as claimed in claim 8, wherein a subjective measurement of wellness based on a contextual knowledge and at least one appropriate weight is assigned to calculate a type of wellness.

12. The system (100) as claimed in claim 8, wherein the root cause of underlying condition in terms of wellness can be identified to provide nudging recommendations to the user.

13. The system (100) as claimed in claim 8, wherein prediction and prognosis of the underlying condition are performed against population in comparison to provide one or more recommendations associated with at least one wellness measures to the user.

14. The system (100) as claimed in claim 8, wherein personalization of wellness through flexibility of defining one or more types of wellness to calculate overall wellness of the user and to provide inputs through longitudinal analysis of specific activity.

15. One or more non-transitory machine-readable information storage mediums comprising one or more instructions which when executed by one or more hardware processors perform actions comprising:

    receiving, plurality of information associated with plurality of sensors;
    processing, the plurality of information associated with the plurality of sensors to obtain a plurality of low-level features;
    determining, at least one digital behavioral marker based on the plurality of low-level features;
    processing, at least one dimension associated with a plurality of well-being of the individual based on the at least one digital behavioral marker to determine a plurality of self-reported analyzed data;
    processing, the at least one dimensions through (i) a plurality of high-level features, and (ii) the plurality of self-reported analyzed data to identify a set of objective features associated with at least one well-being dimension of the individual;
    generating, one or more wellness scores for the set of objective features associated with the at least one well-being dimension of the individual; and
    dynamically recommending, one or more behavior changes based on the one or more generated wellness scores of the individual.

SYSTEM
100

MEMORY
102

DATABASE
108

HARDWARE
PROCESSOR(S)
104

INTERFACE(S)
106

FIG. 1

FIG. 2

receiving, via one or more hardware processors, plurality of information associated with plurality of sensors 302

processing, via the one or more hardware processors, the plurality of information associated with the plurality of sensors to obtain a plurality of low-level features 304

determining, via the one or more hardware processors, at least one digital behavioral marker based on the plurality of low-level features 306

processing, via the one or more hardware processors, at least one dimension associated with a plurality of well-being of the individual based on the at least one digital behavioral marker to determine a plurality of self-reported analyzed data 308

processing, via the one or more hardware processors, the at least one dimensions through (i) a plurality of high-level features, and (ii) the plurality of self-reported analyzed data to identify a set of objective features associated with at least one well-being dimension of the individual 310

generating, via the one or more hardware processors, one or more wellness scores for the set of objective features associated with the at least one well-being dimension of the individual 312

dynamically recommending, via the one or more hardware processors, one or more behavior changes based on the one or more generated wellness scores of the individual 314

FIG. 3

Europäisches
Patentamt

European
Patent Office

Office européen
des brevets

# EUROPEAN SEARCH REPORT

Application Number

EP 21 16 1553

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/125481 A1 (UTTER II MAX EVERETT [US]) 8 May 2014 (2014-05-08) * abstract; figures 3-5e, 12a-b, 15a-17b * * paragraph [0038] * * paragraph [0057] - paragraph [0070] * * paragraph [0077] - paragraph [0081] * * paragraph [0092] - paragraph [0109] * ----- | 1-15 | INV. G16H20/70 G16H50/20 G16H50/30 |
| X | US 2017/300655 A1 (LANE CHRISTINA [CA] ET AL) 19 October 2017 (2017-10-19) * paragraph [0008] - paragraph [0011] * * paragraph [0048] - paragraph [0065] * * paragraph [0152] - paragraph [0158] * ----- | 1-15 | |

TECHNICAL FIELDS SEARCHED (IPC)

G16H

The present search report has been drawn up for all claims

| Place of search | Date of completion of the search | Examiner |
|---|---|---|
| Munich | 29 July 2021 | Menschner, Philipp |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 16 1553

This annex lists the patent family members relating to the patent documents cited in  the above-mentioned European search report.
The members are as contained in the European Patent Office EDP file on
The European Patent Office is in no way liable for these particulars which are merely given for the purpose of information.

29-07-2021

| Patent document cited in search report | | Publication date | Patent family member(s) | | Publication date |
|---|---|---|---|---|---|
| US 2014125481 | A1 | 08-05-2014 | NONE | | |
| US 2017300655 | A1 | 19-10-2017 | CA | 3021230 A1 | 26-10-2017 |
| | | | EP | 3446247 A1 | 27-02-2019 |
| | | | SG | 11201809200R A | 29-11-2018 |
| | | | US | 2017300655 A1 | 19-10-2017 |
| | | | WO | 2017181278 A1 | 26-10-2017 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202021010921